# EUROPEAN PATENT APPLICATION

(11) **EP 2 289 419 A1**
(43) Date of publication of application: **02.03.2011**
(21) Application number: 09746606.4
(22) Date of filing: 13.05.2009
(51) Int. Cl.: A61B 8/00, H04R 19/00, H04R 31/00

(54) **ULTRASONIC PROBE, METHOD FOR MANUFACTURING THE SAME AND ULTRASONIC DIAGNOSTIC DEVICE**

(30) Priority: 15.05.2008 JP 2008128234
(71) Applicant: Hitachi Medical Corporation, Chiyoda-ku Tokyo 101-0021 (JP)
(72) Inventor: SANO, Shuzo, Tokyo 101-0021 (JP); YOSHIMURA, Yasuhiro, Hitachinaka-shi Ibaraki 312-0034 (JP); NAGATA, Tatsuya, Hitachinaka-shi Tochigi 312-0034 (JP); FUKADA, Makoto, Tokyo 101-0021 (JP); SAKO, Akifumi, Tokyo 101-0021 (JP)
(74) Representative: MERH-IP Matias Erny Reichl Hoffmann
(86) International application number: PCT/JP2009/058878
(87) International publication number: WO 2009/139400

(57) **Abstract**

An ultrasonic probe is provided with a CMUT chip having a plurality of transducer elements that change electromechanical coupling coefficients or sensitivities in accordance with a bias voltage to transmit and receive ultrasonic waves, an electric conducting layer formed on the ultrasonic irradiation side of the CMUT chip, an acoustic lens arranged on the ultrasonic irradiation side of the CMUT chip, an insulating layer formed in the direction opposite to the ultrasonic irradiation side of the acoustic lens, a housing unit that stores the CMUT chip in which the electric conducting layer and the insulating layer are fixed with an adhesive and the acoustic lens, wherein the insulating layer is formed by the material that includes at least either silicon oxide or paraxylene to prevent a solvent of the adhesive from soaking into the adhered portion.

## Description

### Field of the Invention

The present invention relates to an ultrasonic probe, method for manufacturing the ultrasonic probe and ultrasonic diagnostic apparatus.

### Description of Related Art

An ultrasonic diagnostic apparatus is for imaging a diagnostic image based on the reflected echo signals outputted from an ultrasonic probe. The ultrasonic probe has a plurality of ultrasonic transducers disposed therein. Ultrasonic transducers convert driving signals into ultrasonic waves to transmit them to an object to be examined, and receive the reflected echo signals produced from the object and convert them into electrical signals.

In recent years, ultrasonic probes using a CMUT (Capacitive Micromachined Ultrasonic Transducer) have been developed. The CMUT is an ultrafine capacity-type ultrasonic transducer manufactured by semiconductor microfabrication process. In CMUT, transmission/reception sensitivity of ultrasonic waves, i.e. electromechanical coupling coefficient varies according to a bias voltage. A bias voltage is applied being overlapped with the driving signal provided from an ultrasonic-wave transmitting/receiving unit (for example, refer to [Patent Document 1]).

### Prior Art Document

### Patent Document

Patent Document 1: US Patent No. 5894452

However, in the CMUT probe disclosed in [Patent Document 1], a DC voltage is applied to the lower electrode as a bias voltage with respect to a silicon substrate. Therefore, there is a need to dispose an insulating layer so as to prevent the part for applying to the object from contacting the upper electrode of the CMUT chip. The insulating layer is formed on an acoustic lens using a method such as vacuum deposition, sputtering or CVD (Chemical Vapor Deposition). On the other hand, an electric conductive layer is formed on the CMUT chip. Then the insulating layer and the electric conducting layer are attached by adhesive agent. Such configured CMUT probe has a problem that when it is immersed in antiseptic solution such as alcohol, the antiseptic solution turns to solvent of adherent agent. The solvent melts adherent agent, and the melted adherent agent penetrate into the CMUT chip. The penetrated adherent agent hardens a frame body and a film body of the CMUT chip, whereby leading to dysfunction in transmission/reception of ultrasonic waves in the inner space segmented by the hardened frame body and film body. Such dysfunction of a CMUT chip caused by penetration of adherent agent still remains unsolved.

The objective of the present invention is to provide the ultrasonic probe, the method for manufacturing the ultrasonic probe and the ultrasonic diagnostic apparatus capable of preventing dysfunction of CMUT chips due to penetration of adherent agent.

### Brief Summary of the Invention

The ultrasonic probe related to the present invention comprises:
a CMUT chip having a plurality of transducers that change electromechanical coupling coefficients or sensitivities in accordance with a bias voltage, configured to transmit/receive ultrasonic waves;
an electric conducting layer formed on the ultrasonic-wave irradiation side of the uMUT chip;
an acoustic lens disposed on the ultrasonic-wave irradiation side of the CMUT chip;
an insulating layer formed in the direction opposite from the ultrasonic-wave irradiation side of the acoustic lens; and
a housing unit configured to store the CMUT chip in which the electric conducting layer and the insulating layer are adhered with adhesive agent and the acoustic lens,
wherein the insulating layer includes at least one of silicon oxide or paraxylene, and is formed by material which prevents the solvent of adhesive agent from penetrating into an adhesive part.

In this manner, it is possible to provide an ultrasonic probe capable of preventing dysfunction of a CMUT chip due to penetration of adhesive agent.

The method of manufacturing the ultrasonic probe related to the present invention comprising:
a CMUT chip having a plurality of transducers that change electromechanical coupling coefficients or sensitivities in accordance with a bias voltage, configured to transmit/receive ultrasonic waves;
an acoustic lens provided on the ultrasonic-wave irradiation side of the CMUT chip;
a backing layer provided on the back surface side of the CMUT chip, configured to absorb transmission of the ultrasonic waves;
an electric wiring unit provided on the side surface of the backing layer from the peripheral border of the CMUT chip, in which the signal pattern connected with an electrode of the CMUT chip is arranged; and
a housing unit configured to store the CMUT chip, the acoustic lens, the backing layer and the electric wiring unit,
is characterized in having:
   a step that adheres the CMUT chip on the top surface of the backing layer;
   a step that adheres the electric wiring unit to the peripheral border of the top surface of the backing layer;
   a step that connects the electric wiring unit and the CMUT chip via a wire;
   a step that fills a sealant around the wire;
   a step that forms electric conducting layer capable of connecting to the ground, on the ultrasonic-wave irradiation side of the CMUT chip; and
   a step that adheres the acoustic lens on the ultrasonic-wave irradiation side of the CMUT chip.

In this manner, it is possible to provide the method for manufacturing an ultrasonic probe capable of preventing dysfunction of a CMUT chip due to penetration of adhesive agent.

The ultrasonic diagnostic apparatus related to the present invention comprises:
an ultrasonic probe configured to transmit/receive ultrasonic waves to/from an object;
an image processing unit configured to construct an ultrasonic image based on the ultrasonic reception signal outputted from the ultrasonic probe; and
a display unit configured to display the ultrasonic image,
wherein the ultrasonic probe is a first ultrasonic probe.

The first ultrasonic probe comprises:
a CMUT chip having a plurality of transducers that change electromechanical coupling coefficients and sensitivities in accordance with a bias voltage, configured to transmit/receive ultrasonic waves;
an electric conducting layer formed on the ultrasonic-wave irradiation side of the CMUT chip;
an acoustic lens disposed on the ultrasonic-wave irradiation side of the CMUT chip;
an insulating layer formed in the direction opposite from the ultrasonic-wave irradiation side of the acoustic lens; and
a housing unit configured to store the CMUT chip in which the electric conducting layer and the insulating layer are adhered with adhesive agent and the acoustic lens,
wherein the insulating layer includes at least one of silicon oxide or paraxylene, and formed by the material capable of preventing penetration of solvent of the adhesive agent into the adhesive agent part.

In this manner, it is possible to provide an ultrasonic diagnostic apparatus capable of preventing dysfunction of a CMUT chip due to penetration of adhesive agent.

In accordance with the present invention, it is possible to provide an ultrasonic probe, the method for manufacturing the ultrasonic probe and ultrasonic diagnostic apparatus capable of preventing dysfunction of a CMUT chip due to penetration of adhesive agent.

### Brief Description of the Diagrams

Fig. 1 is a configuration diagram of ultrasonic diagnostic apparatus 1.
Fig. 2 is a configuration diagram of ultrasonic probe 2.
Fig. 3 is a configuration diagram of transducer 21.
Fig. 4 is a configuration diagram of transducer element 28.
Fig. 5 shows ultrasonic probe 2a related to first embodiment 1.
Fig. 6 is a pattern diagram showing the connection between ultrasonic diagnostic apparatus 1 and ultrasonic probe 2.
Fig. 7 shows ultrasonic probe 2a related to second embodiment.
Fig. 8 shows wiring of ultrasonic probe 2.
Fig. 9 shows ground connection of base plate 40 in CMUT chip 20.
Fig. 10 shows manufacturing process of ultrasonic probe 2 illustrated in Fig. 5.
Fig. 11 shows ultrasonic probe 2f related to fifth embodiment.
Fig. 12 is a detailed drawing of an electric cable illustrated in Fig. 11.
Fig. 13 shows ground connection of base plate 40 viewed from the top-surface side of CMUT chip 20.
Fig. 14 shows ground connection of base plate 40 viewed from the under-surface side of CMUT chip 20.
Fig. 15 is a schematic cross-sectional view showing ultrasonic probe 2c related to a seventh embodiment.
Fig. 16 is a top view of a CMUT wafer related to an eighth embodiment.
Fig. 17 is a top view of CMUT chip related to the eighth embodiment.
Fig. 18 shows the method for forming an electric conducting layer related to a ninth embodiment.
Fig. 19 shows the method for forming an electric conducting layer related to a tenth embodiment.

### Detailed Description of the Invention

Preferable embodiments of the ultrasonic probe and ultrasonic diagnostic apparatus related to the present invention will be described below referring to the attached diagrams. In the following description, the same function parts are represented by the same reference numerals, and the duplicative description thereof is omitted.

### (1. Configuration of the Ultrasonic Diagnostic Apparatus)

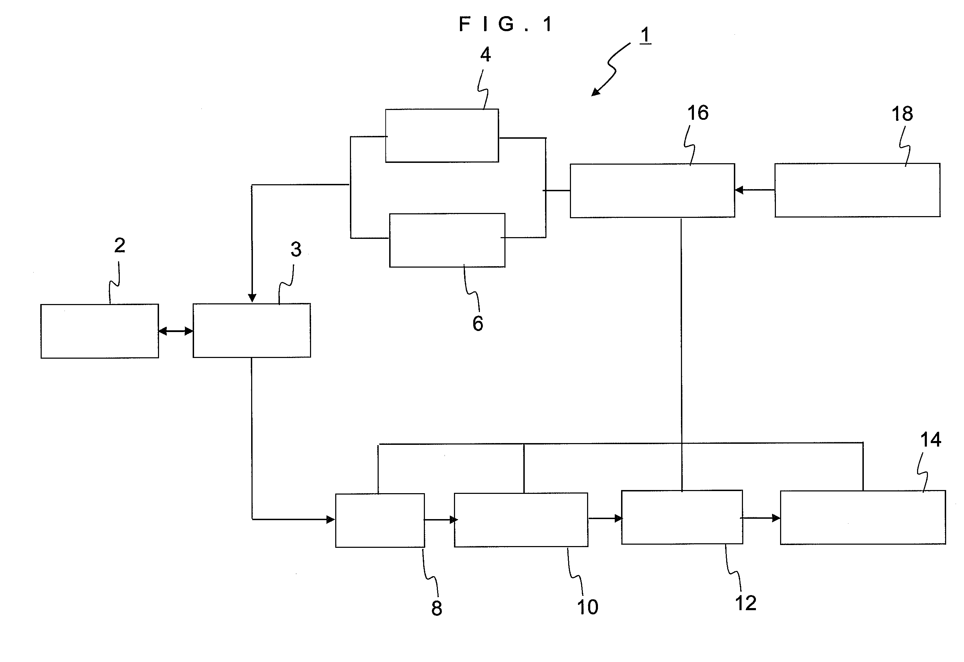

First, configuration of ultrasonic diagnostic apparatus 1 will be described referring to Fig. 1.
Fig. 1 is a configuration diagram of ultrasonic diagnostic apparatus 1.

Ultrasonic diagnostic apparatus 1 is configured by ultrasonic probe 2, transmission/reception separating unit 3, transmitting unit 4, bias unit 6, receiving unit 8, phasing and adding unit 10, image processing unit 12, display unit 14, control unit 16 and operation unit 18.

Ultrasonic probe 2 transmits/receives ultrasonic waves to/from an object to be examined while being applied to the object. Ultrasonic waves are transmitted to the object from ultrasonic probe 2, and the reflected echo signals from the object are received by ultrasonic probe 2.

Transmitting unit 4 and bias unit 6 are the devices that provide driving signals to ultrasonic probe 2.

Receiving unit 8 receives the reflected echo signals outputted from ultrasonic probe 2. Receiving unit 8 further executes processes such as analogue digital conversion to the received reflected echo signals.

Transmission/reception separating unit 3 switches and separates the transmission and reception of ultrasonic waves so as to send the driving signals from transmitting unit 4 to ultrasonic probe 2 upon transmission, and to send the receiving signals from ultrasonic probe 2 to receiving unit 8.

Phasing and adding unit 10 executes phasing and adding of the received reflected echo signals.

Image processing unit 12 constructs a diagnostic image (for example, a tomographic image or blood flow image) based on the phased and added reflected echo signals.

Display 18 displays the image processed diagnostic image.

Control unit 16 controls the above-mentioned respective components.

Operation unit 18 gives commands to control unit 16. Operation unit 18 is an input device such as a trackball, keyboard or mouse.

### (2. Ultrasonic probe 2)

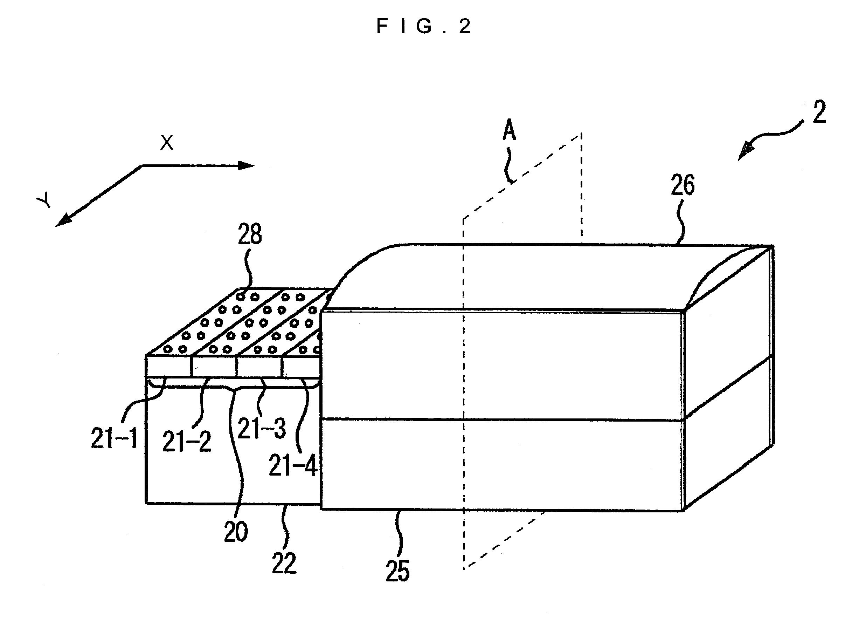

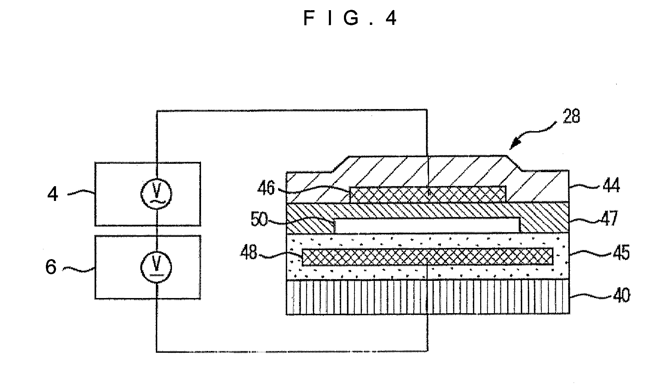

Next, ultrasonic probe 2 will be described referring to Fig. 2 ∼ Fig. 4.

### (2-1. Configuration of Ultrasonic Probe 2)

Fig. 2 is a configuration diagram of ultrasonic probe 2. Fig. 2 is a partially-notched perspective view of ultrasonic probe 2.

Ultrasonic probe 2 comprises CMUT chip 20. CMUT chip 20 is a group of one-dimensional array type transducers in which a plurality of transducers 21-1, 21-2, ... are disposed in strips. In transducer 21-1, transducer 21-2, ..., a plurality of transducer elements 28 are disposed. Other types of transducer group such as the 2-dimensional array type or convex type may also be used instead.

Backing layer 22 is provided on the back-surface side of CMUT chip 20. Acoustic lens 26 is provided on the ultrasonic-wave irradiation side of CMUT chip 20. CMUT chip 20 and backing layer 22, etc. are contained in ultrasonic probe cover 25.

CMUT chip 20 converts the driving signals from transmitting unit 4 and bias unit 6 into ultrasonic waves, and transmits the ultrasonic waves to the object. Receiving unit 8 converts the ultrasonic waves produced from the object into electronic signals, and receives them as reflected echo signals.

Backing layer 22 absorbs the transmission of ultrasonic waves projected from CMUT chip 20 to the back-surface side for suppressing superfluous vibration.

Acoustic lens 26 converges the ultrasonic beams transmitted from CMUT chip 20. Curvature of acoustic lens 26 is specified based on one focal distance.

A matching layer may also be provided between acoustic lens 26 and CMUT chip 20. The matching layer interfaces CMUT chip 20 and acoustic impedance of the object whereby improving transmission efficiency of ultrasonic waves.

### (2-2. Transducer 21)

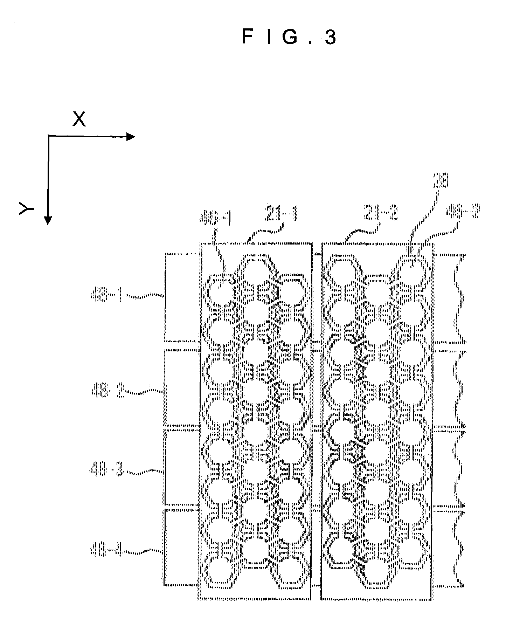

Fig. 3 is a configuration diagram of transducers 21.

Upper electrode 46 of transducer element 28 is connected for each transducer 21 sectioned in major-axis direction X. In other words, upper electrode 46-1, upper electrode 46-2, ... are juxtaposed in major-axis direction X.

Lower electrode 48 of transducer element 28 is connected for each transducer 21 sectioned in minor-axis direction Y. In other words, upper electrode 48-1, upper electrode 48-2, ... are juxtaposed in minor-axis direction Y.

### (2-3. Transducer Element 28)

Fig. 4 is a configuration diagram of transducer element 28. Fig. 4 is a cross-sectional view of one transducer element 28.

Transducer element 28 is configured by base plate 40, film body 44, film body 45, upper electrode 46, frame body 47 and lower electrode 48. Transducer element 28 is formed by semiconductor microfabrication process. Transducer element 28 is equivalent to one element of a CMUT.

Base plate 40 is a semiconductor substrate such as silicon.
Film body 44 and frame body 47 are formed by semiconductor compound such as silicon compound. Film body 44 is provided on the ultrasonic-wave irradiation side of frame body 47. Upper electrode 46 is provided between film body 44 and frame body 47. Lower electrode 48 is provided on film body 45 formed on base plate 40. Inner space 50 sectioned by frame body 47 and film body 45 is vacuated or filled with a predetermined gas.

Upper electrode 46 and lower electrode 48 are connected to transmitting unit 4 for providing AD high-frequency voltage as a driving signal and bias unit 6 for applying AD voltage as a bias voltage respectively.

When an ultrasonic wave is transmitted, AD bias voltage (Va) is applied to transducer element 28 via upper electrode 46 and lower electrode 48, and an electric field is generated by bias voltage (Va). Film body 44 is under tension due to the generated electric field and has a predetermined electromechanical coupling coefficient (Sa). When a driving signal is provided from transmitting unit 4 to upper electrode 46, an ultrasonic wave is projected from film body 44 based on the electromechanical coupling coefficient (Sa).

Also, when AD bias voltage (Vb) is applied to transducer element 28 via upper electrode 46 and lower electrode 48, an electric field is generated by bias voltage (Vb). Film body 44 is under tension due to the generated electric field, and has predetermined electromechanical coupling coefficient (Sb). When a driving signal is provided from transmitting unit 4 to upper electrode 46, an ultrasonic wave is projected based on electromechanical coupling coefficient (Sb).
Here, when bias voltage is "Va < Vb", electromechanical coupling coefficient is "Sa < Sb".

On the other hand, in the case of receiving an ultrasonic wave, film body 44 is excited by the reflected echo signal generated from the object, and capacity of inner space 50 changes. According to the variation of inner space 50, an electric signal is detected via upper electrode 46.

Electromechanical coupling coefficient of transducer element 28 is determined by the tension of film body 44. Therefore, by controlling the tension of film body 44 by changing the bias voltage to be applied to transducer element 28, even when the driving signal of the same amplitude is inputted, acoustic pressure (for example, amplitude) of the ultrasonic wave projected from transducer element 28 can be changed.

### (3. First Embodiment)

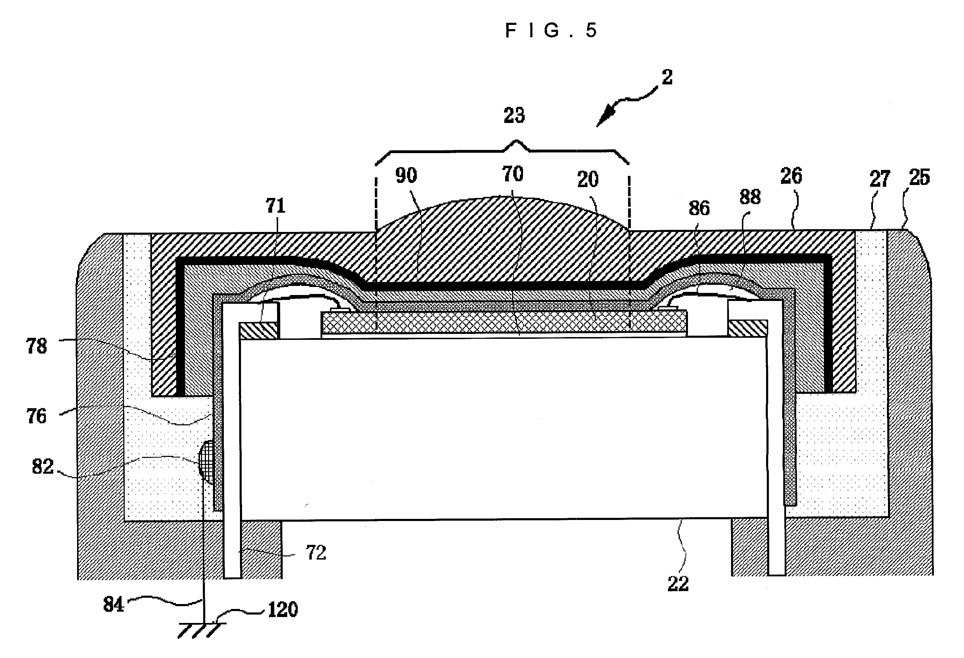

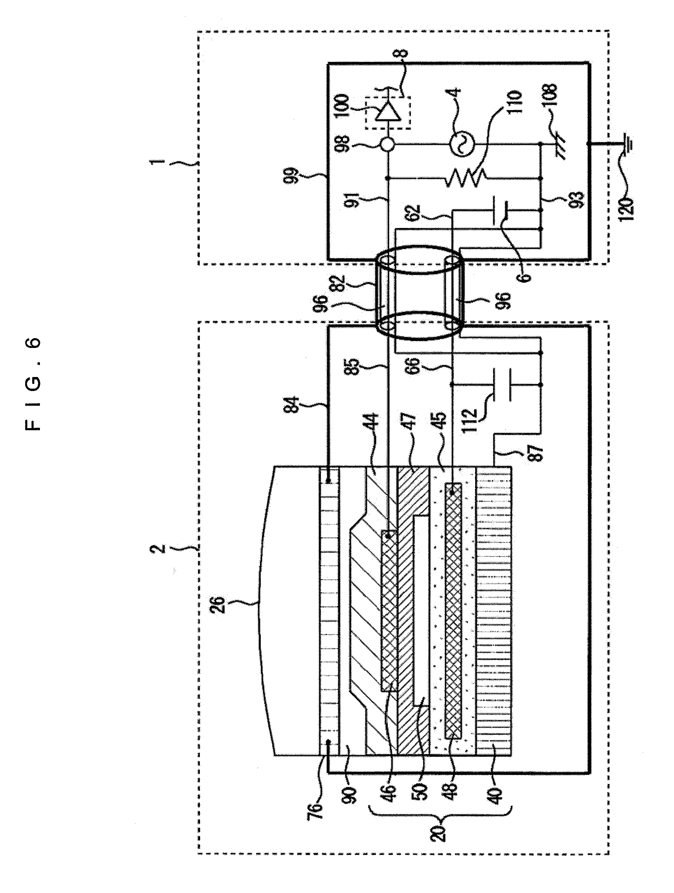

Next, the first embodiment will be described referring to Fig. 5 and Fig. 6.

### (3-1. Configuration Member of Ultrasonic Probe 2)

Fig. 5 shows ultrasonic probe 2 related to the first embodiment. Fig. 5 is a cross-sectional view of plane A of ultrasonic probe 2 shown in Fig. 2.

Electric conducting layer 76 is formed along the ultrasonic-wave irradiation side of CMUT chip 20 and the side surface of flexible substrate 72 and backing layer 22, and insulating film 78 which is an insulating layer is formed on the inner surface of acoustic lens 26. Electric conducting layer 76 and insulating layer 78 are formed by the method such as vacuum deposition, sputtering or CVD, and electric conducting layer 76 is formed by Cu or AI film having electrical conductivity. Insulating layer 78 is attached to electric conducting layer 76 by adhesive. Insulating layer 78 is formed by silicon oxide film or paraxilene film, etc. and is chemical resistant. Being chemical resistant here means that is to suppress penetration of, for example, adherent used for adhering acoustic lens 26 into CMUT chip 20. Electric conducting layer 76 is connected to ground 120 which is on the side of a main body via terminal area 82 connected by soldering or conductive adhesive and ground 84.

In this manner, since electric conducting layer 76 is provided on the ultrasonic-wave irradiation side of CMUT chip 20 as the ground layer, electrical safety of ultrasonic probe 2 with respect to the object can be improved. Also, since insulating film 78 is formed as insulating layer between acoustic lens 26 and CMUT chip 20, the space between acoustic lens 26 and CMUT chip 20 can be protected by double insulation by acoustic lens 26 and insulating layer 78. Therefore, safety of ultrasonic probe 2 can be improved. By providing more than two layers of insulating layers rather than one layer of insulating layer 78, electrical safety can be further improved.

Since electric conducting layer 76 is formed on the ultrasonic-wave irradiation side of CMUT chip 20, it is not necessary to form the electrical conducting layer on the inner side of acoustic lens 26. Also, since electric conducting layer 76 is formed along the side surface of flexible substrate 72 and backing layer 22, electric conducting layer 76 and ground wire 84 can be connected directly via terminal area 82 by having backing layer 22 as the base.

CMUT chip 20 is adhered on the top surface of backing layer 22 via adhesive layer 70. Flexible substrate 72 (Flexible Printed Circuits: FPC) is provided along the peripheral border of the top surface and the side surfaces in four-directions of backing layer 22. Flexible substrate 72 is adhered on the peripheral border of the top surface of backing layer 22 via adhesive layer 71.

Adhesive layer 70 and adhesive layer 71 are made of, for example, epoxide resin. By arbitrarily adjusting the thickness of adhesive layer 70 and adhesive layer 71, the height and directional position of CMUT chip 20 and flexible substrate 72 can be adjusted.

Flexible substrate 72 and CMUT chip 20 are electrically connected via wire 86. Wire 86 is connected using the wire-bonding method. As for wire 86, an Au wire, etc. can be used. Around the wire 86, material such as photo curing resin 88 is filled as a sealant. Heat-hardening resin also may be used as the sealant. As for the thermal-hardening resin, the material having the same thermal expansion coefficient as the base material of the semiconductor should be used. By using thermal-hardening resin, material strength by thermal expansion can be improved more than the case of using photo curing resin. In place of the wire-bonding method, the flip-chip bonding method which enables attachment between pads may be used. By using the flip chip bonding method, limit in mounting such as chip size can be modified, compared to the internal connection by the wire bonding method.

Acoustic lens 26 is adhered to CMUT chip 20 via adhesive layer 90 between insulating layer 78 formed on the inner surface and electric conducting layer 76 formed on the ultrasonic-wave irradiation side of CMUT chip 20. As for the material for acoustic lens 26, for example, silicon rubber is used. As for the material for adhesive layer 90, it is desirable to use a material similar to the adhesive layer used for acoustic lens 26 (for example, silicon resin).

The ultrasonic-wave irradiation surface of acoustic lens 26 is convex in the ultrasonic-wave irradiating direction within the range of at least region 23. In CMUT chip 20, transducer element 28 is disposed within the range corresponding at least to region 23. Ultrasonic waves are irradiated from the convex part of acoustic lens 26.

The back surface of acoustic lens 26 has a concave portion at the position corresponding to the peripheral border of CMUT chip 20. In this concave portion, a terminal area (the part of photo curing resin 88) of CMUT chip 20 and flexible substrate 72 is fitted.

Ultrasonic probe cover 25 is provided to the side surfaces in the four direction of ultrasonic probe 2. Ultrasonic probe cover 25 is fixed on the side surfaces in the four directions of acoustic lens 26. An examiner operates ultrasonic probe 2 by holding ultrasonic probe cover 25 with his/her hand. Sealant 27 is filled in the gap between ultrasonic probe cover 25 and acoustic lens 26.

It is desirable that the upper end of ultrasonic probe cover 25 is positioned in the part above CMUT chip 20. In this manner, even in an unexpected contingency such as dropping of the probe should occurred, CMUT chip 20 can be protected by restraining direct impact shock.

### (3-2. Connection of Ultrasonic Probe 2)

Fig. 6 is a pattern diagram showing the connection between ultrasonic diagnostic apparatus 1 and ultrasonic probe 2. Ultrasonic diagnostic apparatus 1 and ultrasonic probe 2 are connected via cable 82. Cable 82 has a plurality of coaxial cables 96.

Upper electrode 46 of transducer 28 is connected to wiring 85. Wiring 85 is connected to wiring 91 in ultrasonic diagnostic apparatus 1 via an inner conductor of coaxial cable 96. Wiring 91 is connected to receiving amplifier 100 in receiving unit 8 and transmitting unit 4 via transmission/reception separating circuit 98.

Lower electrode 48 of transducer element 28 is connected to wiring 66. Wiring 66 is connected to wiring 62 in ultrasonic diagnostic apparatus 1 via an inner conductor of coaxial cable 96. Wiring 62 is connected to bias unit 6.
The number of coaxial cables 36 is to be the sum of upper electrodes 46 and lower electrodes 48 commonly disposed in a plurality of transducer elements 28.

Substrate 40 of transducer element 28 is connected to wiring 87. Wiring 87 is connected to wiring 93 of ultrasonic diagnostic apparatus 1 via an outer conductor of coaxial cable 96. Wiring 93 is connected to ground 108 via a chassis ground of the main body (not shown in the diagram).

Condenser 112 is disposed between wiring 66 and wiring 87. This condenser 112 is a capacitative element for bypassing the electrical current from lower electrode 48.

Resistor 110 is disposed between wiring 91 and wiring 93. This resistor 110 is a resistance element for stabilizing the DC potential of upper electrode 46 as the ground electrode in the case that AC current flows from upper electrode 46 to lower electrode 48.

Bias unit 6 is disposed between wiring 62 and wiring 93. This bias unit 6 is for generating electric potential difference between upper electrode 46 and lower electrode 48. Also, transmitting unit 4 causes upper electrode 46 to apply an AD high-frequency voltage as a driving signal. Concretely, DC=ground (reference potential) and AC=Vpp in upper electrode 46, and DC=Vdc and AC=0 in lower electrode 48.

Electric conducting layer 76 of transducer element 28 is connected to wiring 84. Wiring 84 is formed to cover inner circuits (wiring 85, wiring 66, condenser 112, etc.) of ultrasonic probe 2, and is connected to wiring 99 in ultrasonic diagnostic apparatus 1 via outer circumference of cable 82. Wiring 99 is formed to cover inner circuits (wiring 91, wiring 62, resistor 110, etc.) of ultrasonic diagnostic apparatus 1, and is connected to ground 120. Therefore, DC=0 and AC=0 in electric conducting layer 76, wiring 84, outer circumference of cable 82 and wiring 99.

Electric conducting layer 76, wiring 84, outer circumference of cable 82, wiring 99 and ground 120 form a protection circuit, prevent penetration of electromagnetic waves from outside into inner circuits of ultrasonic diagnostic apparatus 1 and ultrasonic probe 2, and also prevent discharge of electricity generated inside of ultrasonic diagnostic apparatus 1 and ultrasonic probe 2 to the outside.

### (3-3. Effect of the First Embodiment)

In this manner, insulating layer 78 is provided on the ultrasonic-wave irradiation side of CMUT chip 20 in ultrasonic probe 2 of the first embodiment. Thus it is possible to prevent dysfunction of CMUT chip due to penetration of adhesive.

Since electric conducting layer 76 is disposed on the ultrasonic-wave irradiation side of CMUT chip 20 and electric conducting layer is a ground potential, it is possible to prevent electrification even when acoustic lens 26 on the irradiation side is damaged, whereby improving electrical safety of the ultrasonic probe with respect to an object.

Also, by electric conducting layer 76, ground wiring 84 and the chassis ground of the main device, an enclosed space of the ground potential is formed. In other words, since the main components or main body circuits of ultrasonic probe 2 are contained in the enclosed space having the ground potential, negative influence due to extraneous electrical waves or the electromagnetic waves produced from ultrasonic probe 2 to the outer device can be prevented.

Also, ultrasonic probe 2 in the first embodiment, electric conducting layer 76 is formed along the inner surface and the outer surface of acoustic lens 26, and is connected to ground 120 via highly-reliable electric conducting member 80 and ground wiring 84.

By such configuration, electric conducting layer 76 formed along the inner and outer surfaces of acoustic lens 26, not the in-mold formed sheet-like electric conducting layer to be pulled out, can be easily and surely connected to ground wire 84 via electric conducting member 80, thereby improving its mounting reliability and workability.

Also, by using highly-reliable electric conducting layer 80, it is possible to prevent electric conducting member 80 from being damaged upon being fixed on flexible substrate 72.

Also, while electric conducting member 80 and ground wire 84 are illustrated only on the left side surface of flexible substrate 72 in Fig. 5, they can be disposed in any one or more side surfaces in four directions of flexible substrate 72.

### (4. Second Embodiment)

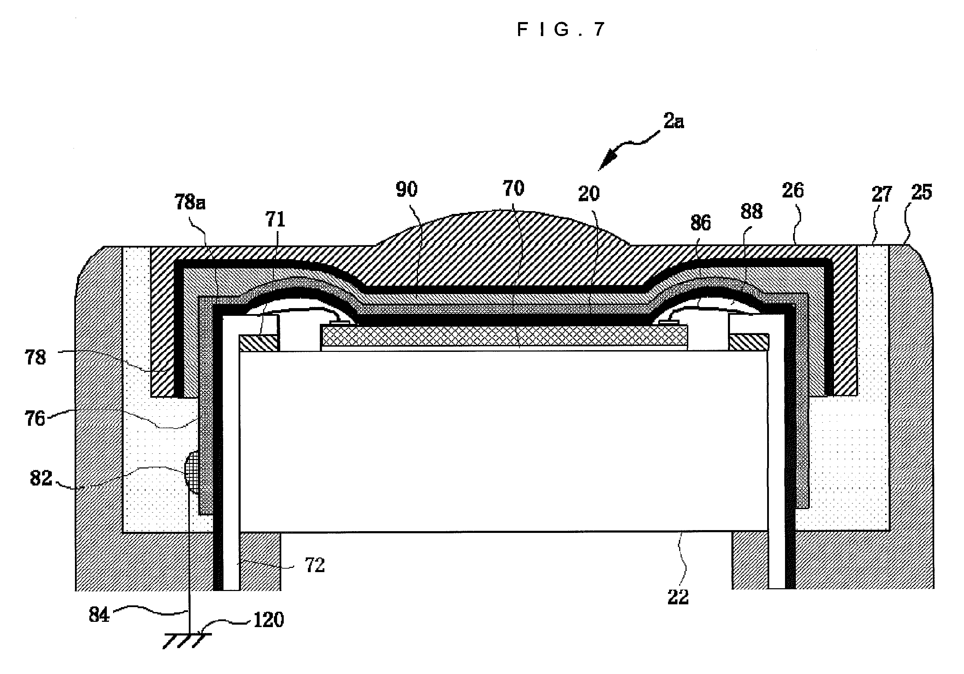

Next, the second embodiment will be described referring to Fig. 7.
Fig. 7 shows ultrasonic probe 2a related to the second embodiment. Fig. 7 is equivalent to the cross-sectional view of plane A in Fig. 2.

While electric conducting layer 76 is formed along the ultrasonic-wave irradiation side of CMUT chip 20 and the side surfaces of flexible substrate 72 and backing layer 22 and connected to ground wire 84 via adhesive portion 82 in the first embodiment, insulating film 78a is to be formed as an insulating layer between CMUT chip 20 and electric conducting layer 76 in the second embodiment. In the same manner as embodiment 1, insulating film 78 is formed as an insulating layer on the inner surface of acoustic lens 26.

In accordance with the second embodiment, dysfunction of CMUT chip due to penetration of adhesive can be prevented as in the first embodiment. Also, since electric conducting layer 76 is provided as the ground layer on the ultrasonic-wave irradiation side of CMUT chip 20, electrical safety of ultrasonic probe 2a can be improved with respect to an object, and voltage endurance can be improved between electric conducting layer 76 and wiring 86 and also between electric conducting layer 76 and CMUT chip 20 by providing insulating film 78a.

### (5. Third Embodiment)

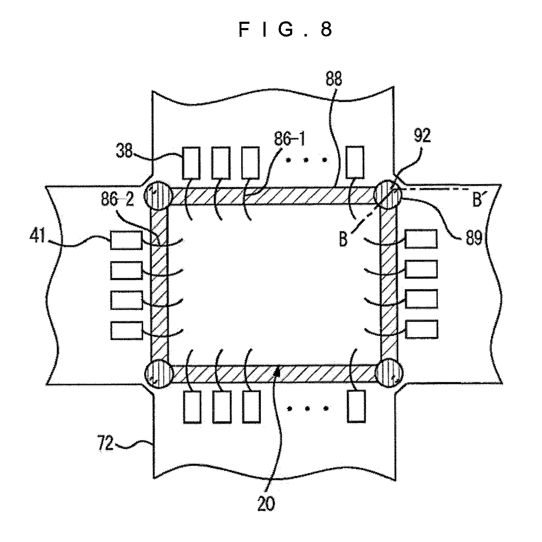

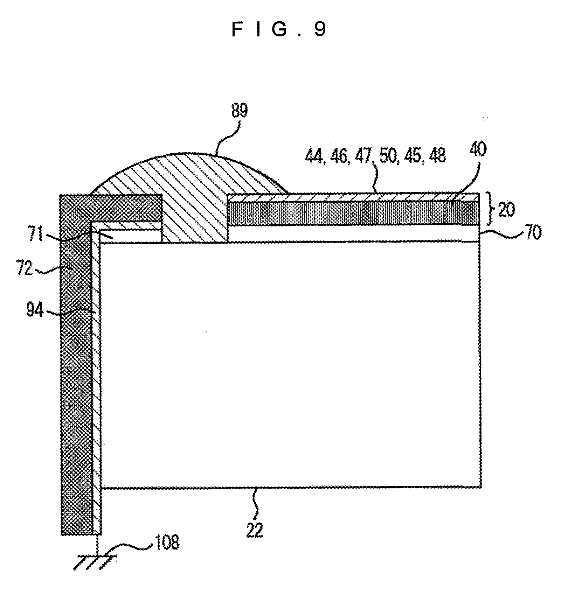

Next, the third embodiment will be described referring to Fig. 8 and Fig. 9.
Fig. 8 is a pattern diagram showing the wiring of ultrasonic probe 2.
Fig. 9 shows the ground connection of base plate 40 in CMUT chip 20, and is the cross-sectional view of the B-B line illustrated in Fig. 8.

In the periphery border of the top surface of CMUT chip 20, upper electrode 46 of CMUT chip 20 and signal pattern 38 of flexible substrate 72 are connected by wire 86-1, and lower electrode 48 of CMUT chip 20 and signal pattern 41 of flexible substrate 72 are connected by wire 86-2. Photo curing resin 88 is filed around wire 86 and the terminal area is sealed.

In a corner portion of CMUT chip 20, electric conducting resin 89 is filled between CMUT chip 20 and flexible substrate 72. Electric conducting resin 89 is equivalent to the terminal area between base plate 40 of CMUT chip 20 and ground wire 94. Ground wire 94 is disposed between flexible substrate 72 and backing layer 22 in the corner portion of CMUT chip 20.

Base plate 40 is provided at the bottom surface of CMUT chip 20. Base plate 40 is electrically connected to electric conducting resin 89. Base plate 40 is connected to ground 108 via electric conducting resin 89 and ground wire 94.
Ground wire 94 in Fig. 9 is equivalent to wiring 87 in Fig. 6. Electric conducting resin 89 is provided to the terminal area between base plate 40 and wiring 87.

In this manner, in accordance with the third embodiment, it is possible to prevent dysfunction of CMUT chip due to penetration of adhesive.

Also, while there is wire 86 for connecting signal pattern 38 and signal pattern 41 of flexible substrate 72 and CMUT chip 20 in the periphery border of CMUT chip except the corner portion, base plate 40 of CMUT chip 20 and ground wire 94 are connected via electric conducting resin 89 which is filled in the corner portion of CMUT chip 20. In this manner, a signal pattern connecting portion and a base plate ground connecting portion can be provided in the separate places, which makes its manufacturing easier.

Since base plate 40 itself is a semiconductor, there is a possibility that high voltage is generated by base plate 40 in abnormal situations. In the third embodiment, base plate 40 can be maintained in the ground potential by ground-connecting base plate 40 even in abnormal situations, to maintain safety of ultrasonic probe 2.

### (6. Fourth Embodiment)

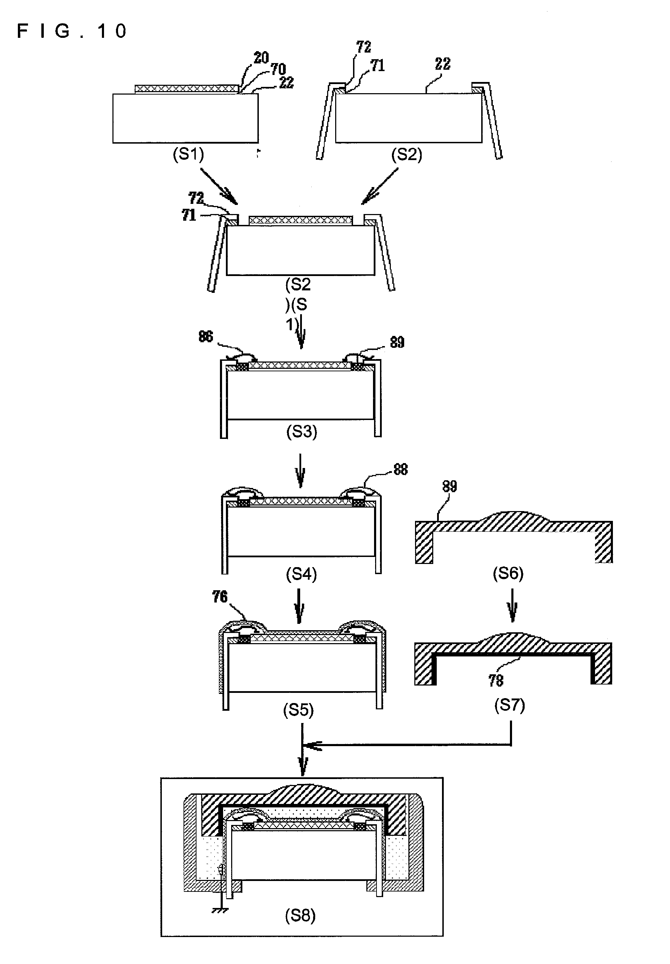

Next, the fourth embodiment will be described referring to Fig. 10. The fourth embodiment relates to the manufacturing method of ultrasonic probe 2 shown in Fig. 5. Fig. 10 shows the manufacturing process of ultrasonic probe 2 illustrated in Fig. 5.

CMUT chip 20 is adhered on the top surface of backing layer 22 by adhesive layer 70 (step S1).
Flexible substrate 72 is adhered to the periphery border of the top surface of backing layer 22 by adhesive 71 (step S2) .
Flexible substrate 72 and CMUT chip 20 are electrically connected via wire 86. Wire 86 is connected using the wire bonding method or flip chip bonding method (step S3).

### Photo curing resin 88 is filled around wire 86 as a sealant (step S4).

Electric conducting layer 76 is formed (step S5).
Acoustic lens 26 is formed (step S6), and insulating layer 78 is formed on the inner surface of acoustic lens 26 (step S7) .

Acoustic lens 26 is adhered to the ultrasonic-wave irradiation surface of CMUT chip 20 by adhesive layer 90. Electric conducting layer 76 is connected to ground wire 84. Ultrasonic probe cover 25 is attached onto an ultrasonic probe. Sealant 27 is filled in the gaps between acoustic lens 26 or flexible substrate 72 and ultrasonic probe cover 25 (step S8).

According to the process described above, ultrasonic probe 2 shown in Fig. 5 is manufactured.
Also, insulating layer 78a may be formed before forming electric conducting layer 76 in the process of step S5. In this case, ultrasonic probe 2a shown in Fig. 7 is manufactured.

As for the forming of a layer, there are methods such as in-mold forming an insulating sheet attached with an electric conducting layer at the same time with the forming of acoustic lens 26, or forming an insulating layer or electric conducting layer by physical or chemical vapor deposition. The in-mold forming method can form a layer with low cost, but the limit in thickness in forming the layer is about 10/µm. On the other hand, the vapor deposition method can form the layer as thin as about 1µm in thickness.

### (7. Fifth Embodiment)

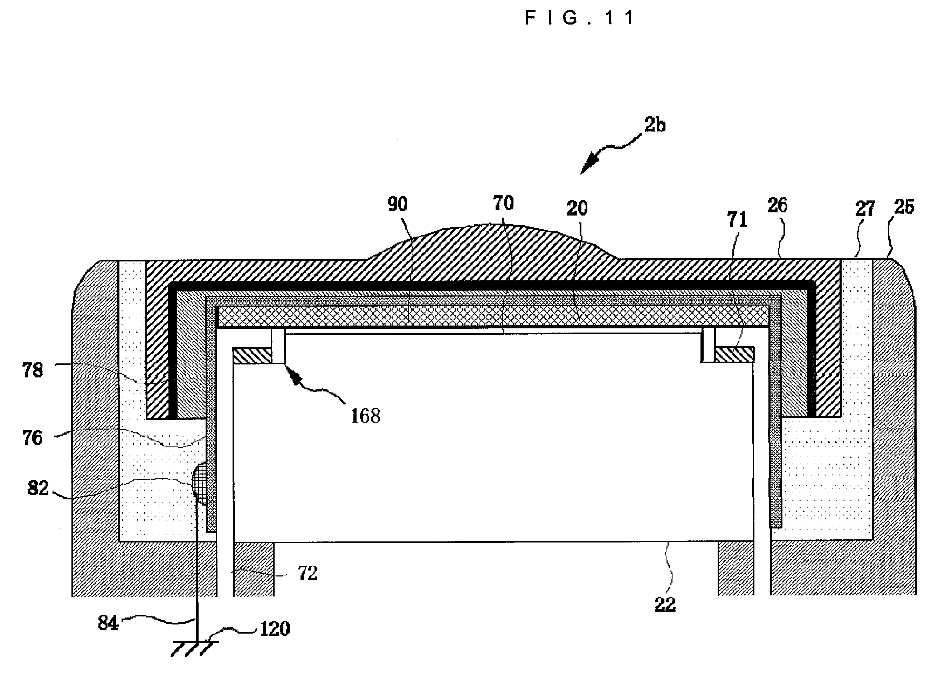

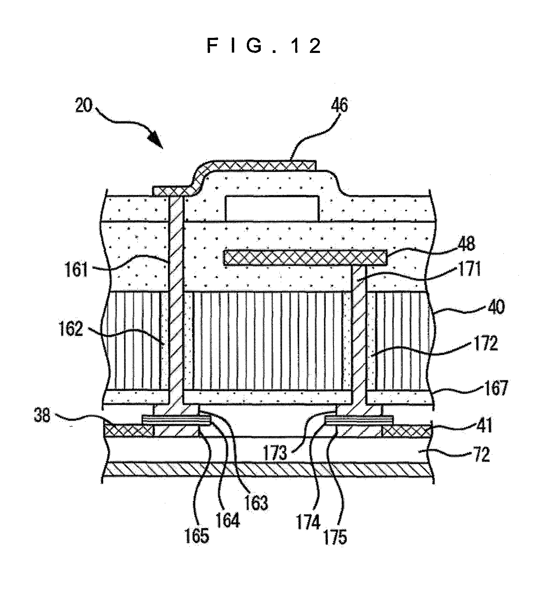

Next, the fifth embodiment will be described referring to Fig. 11 and Fig. 12. The fifth embodiment relates to the electrical connection between CMUT chip 20 and flexible substrate 72.

Fig. 11 shows ultrasonic probe 2f related to the fifth embodiment. Fig. 11 is equivalent to the cross-sectional view of plane A in Fig. 2.
Fig. 12 is a detailed diagram of the electric terminal area shown in Fig. 11.

While flexible substrate 72 and CMUT chip 20 are electrically connected via wire 86 using wire bonding method in the first embodiment, they are electrically connected via through hole 161 or through hole 171 in the fifth embodiment.

The signal pattern of flexible substrate 72 is electrically connected with the electrode of CMUT chip 20 in the periphery border portion of the back surface of CMUT chip 20. In the electric terminal area, notch portion 168 is provided on the periphery border portion of the top surface of backing layer 22 according to the thickness of flexible substrate 72, adhesive layer 71 and adhesive layer 70.

Through hole 161 is a conducting path between upper electrode 46 of CMUT chip 20 and pad terminal 163 provided on the back surface of CMUT chip 20. Through hole 171 is a conducting path between lower electrode 48 of CMUT chip 20 and pad terminal 173 provided on the back surface of CMUT chip 20.

In through hole 161 and through hole 171, metal is filled or a metal layer is formed on their inner wall. In the part of base plate 40 in CMUT chip 20, insulating portion 162 and insulating portion 172 are provided around through hole 161 and through hole 171. It is preferable to provide insulating portion 167 also on the back surface of base plate 40.

Pad terminal 165 and pad terminal 175 provided to flexible substrate 72 are electrically connected to pad terminal 163 and pad terminal 173 provided to the under surface of CMUT chip 20 respectively by electric conducting adhesive 164 and electric conducting adhesive 174 such as anisotropic conducting adhesive sheets.

Signal pattern 38 of flexible substrate 72 is electrically connected to upper electrode 46 of CMUT chip 20 via pad terminal 165, electric conducting adhesive 164, pad terminal 163 and through hole 161. Signal pattern 41 of flexible substrate 72 is electrically connected to lower electrode 48 of CMUT chip 20 via pad terminal 175, electric conducting adhesive 174, pad terminal 173 and through hole 171.

In this manner, in the fifth embodiment, flexible substrate 72 and CMUT chip 20 are electrically connected via through hole 161 and through hole 171. By such configuration, flexible substrate 72 and CMUT chip 20 can be electrically connected only by positioning the pad terminals without wires for electrical connection.

While electrical connection is executed via a through hole on the back surface of CMUT chip in Fig. 12, it may be executed via a through hole on the ultrasonic-wave irradiation side of CMUT chip 20.

Also, in the case of connecting the electrode of CMUT chip 20 and the signal wire of flexible substrate 72 using the wire bonding method illustrated in Fig. 5, since wire 86 having high potential and electric conducting layer 76 having ground potential come close to each other, there are cases that ground potential of electric conducting layer 76 cannot be maintained since short-circuit is caused between electric conducting layer 76 and wire 86 due to defect of sealant such as photo curing resin 88 or pinhole defect of insulating layer 78. On the other hand, in the case that the electrode of CMUT chip 20 and the signal wire of flexible substrate 72 are connected by the through hole shown in Fig. 11 and Fig. 12, since the connecting wire and electric conducting layer 76 are not close to each other, short-circuit can be prevented and the ground potential of electric conducting layer 76 can be maintained, which leads to improvement in safety.

Also, since wire 86 used in the wire bonding method shown in Fig. 5 is a thin metal wire, it is subject to damage by acting force thus difficult to handle. On the other hand, in connection by the through holes shown in Fig. 11 and Fig. 12, the process for connecting wires in the wire bonding method is not necessary, which makes it easier to handle.

Also, in the connection using the wire bonding method shown in Fig. 5, etc., a sealant such as photo curing resin 88 is necessary for filling around wire 86. The resin used as a sealant and wire 86 have different linear expansion coefficients. Generally, the linear expansion coefficient of the resin to be used for a sealant is greater than the one of metal. Thus alteration of temperature caused by expansion of the resin used for a sealant could lead to damage of wire 86. Also, in the case that impure substance is included in the resin used for a sealant, electrical migration could lead to short-circuit between wire 86 and electric conducting layer 76. On the other hand, in the connection using through holes shown in Fig. 11 and Fig. 12, problems due to impure substances included in the resin will not be posed since wires or sealant are not necessary.

In this manner, in the fifth embodiment, by executing connection using through holes in place of the connection by the wire bonding method, safety of ultrasonic probe 2 can be improved.

### (8. Sixth Embodiment)

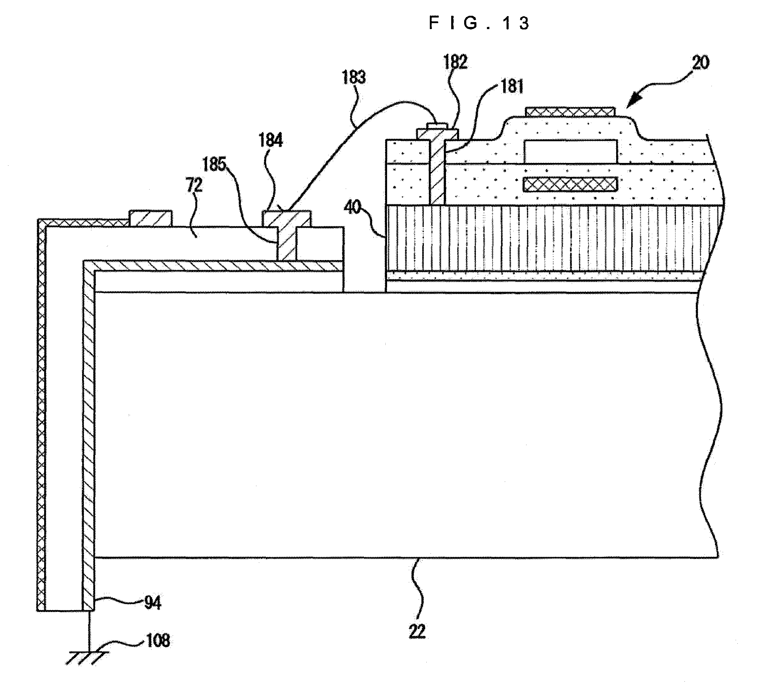

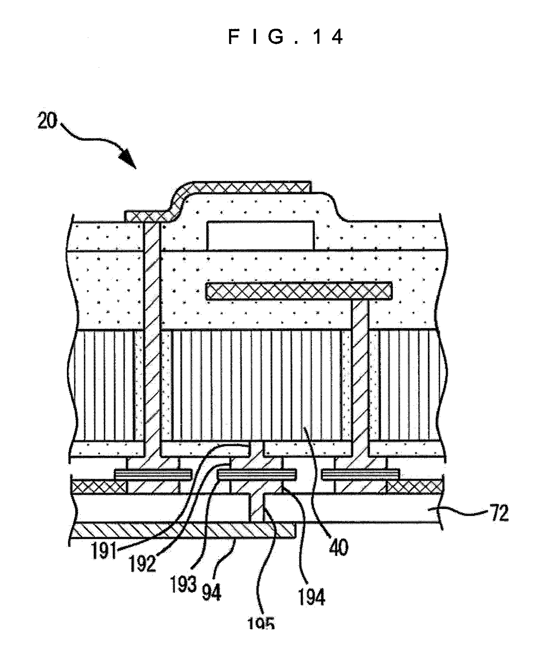

Next, the sixth embodiment will be described referring to Fig. 13 and Fig. 14. The sixth embodiment relates to the ground connection of substrate 40 in CMUT chip 20.
While substrate 40 is ground-connected via electric conducting resin 89 from the side surface of CMUT chip 20 in the third embodiment, substrate 40 is to be ground-connected from the top surface side (ultrasonic-wave irradiation side) or the lower surface side (back surface side) of CMUT chip 20 in the sixth embodiment.

### (8-1. Ground connection from top surface side of CMUT chip)

Fig. 13 shows the ground connection of substrate 40 from the top surface side of CMUT chip 20.
Through hole 181 is a conducting path between substrate 40 of CMUT chip 20 and pad terminal 182 provided on the top surface of CMUT chip 20. Through hole 185 is a conducting path between ground wire 94 provided on the inner surface of flexible substrate 72 and pad terminal 184 provided on the top surface. Through hole 181 and through hole 185 are filled with metal, or a metal layer is formed on the inner walls thereof.

Pad terminal 182 and pad terminal 184 are electrically connected via wire 183 using the wire bonding method. Substrate 40 of CMUT chip 20 is connected to ground 108 via through hole 181, pad terminal 182, wire 183, pad terminal 184, through hole 185 and ground wire 94.

### (8-2. Ground connection from bottom surface side of a CMUT chip)

Fig. 14 shows the ground connection of substrate 40 from the bottom surface side of CMUT chip 20.
Through hole 191 is a conducting path between substrate 40 of CMUT chip 20 and pad terminal 192 provided to the bottom surface of CMUT chip 20. Through hole 195 is a conducting path between ground wire 94 provided on the inner surface of flexible substrate 72 and pad terminal 194 provided on the top surface. Through hole 191 and through hole 195 are filled with metal, or a metal layer is formed on the inner walls thereof.

Pad terminal 192 and pad terminal 194 are electrically connected by electric conducting adhesive 193 such as an anisotropic conducting adhesive sheet 193. Substrate 40 of CMUT chip 20 is ground connected via through hole 191, pad terminal 192, electric conducting adhesive 193, pad terminal 194, through hole 195 and ground wire 94.

### (8-3 Effect of Sixth Embodiment)

In this manner, in the sixth embodiment, substrate 40 of CMUT chip 20 can be ground-connected from the top surface or the bottom surface of CMUT chip 20 via through holes. By such configuration, ground connection of substrate 40 in CMUT chip 20 can be executed only by implementing the connection using the wire bonding method or positioning of the pad terminals, as substitute for filling electric conducting resin for ground-connection. By setting substrate 40 as the ground potential, the electric potential of CMUT chip can be stabilized which leads to stabilization of ultrasonic characteristics.

In addition, there are upper electrode 46 and lower electrode 48 to which more than 100V of high voltage is applied, on substrate 40 of CMUT chip 20. Since substrate 40 itself is a semiconductor, there is a possibility that substrate 40 will have a high voltage in abnormal circumstances. In the sixth embodiment, it is possible to maintain substrate 40 in the ground potential even in abnormal circumstances by ground-connecting substrate 40 via through holes, which leads to improvement in safety of ultrasonic probe 2.

### (9. Seventh Embodiment)

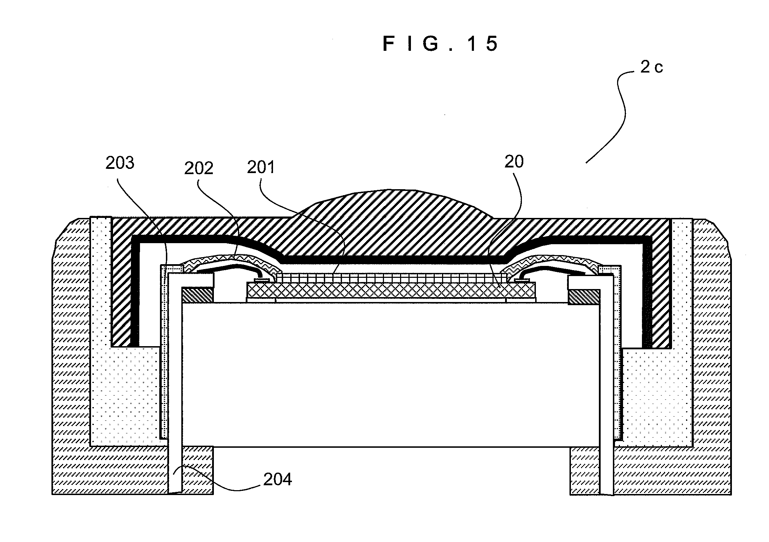

Next, the seventh embodiment will be described referring to Fig. 15.
Electric conducting layer 201 is formed in CMUT chip 20, and it is formed in the process of manufacturing the CMUT wafer before segmentizing it into a number of CMUT chips. Since the CMUT wafer is manufactured using the semiconductor processing, this electric conducting layer 201 is formed in the wafer condition. While Al, Al-Cu alloy or Cu is used for the electric conducting layer in the above-mentioned semiconducting process, electrically conductive substance such as Ti, Cr, Au, Pt, TiN, TiW, or Si3N4 can be applied.

As for the layer manufacturing method, there are methods such as vacuum deposition, sputtering and CVD. Also, while it is possible to form the above-mentioned electric conducting layer 201 after completing the manufacturing process of the CMUT wafer, it is preferable to form electric conducting layer 201 in the wafer condition before segmentizing the CMUT wafer using a method such as dicing, so as to avoid remnant of extraneous substances. In this case, electric conducting layer 201 can be formed using the method to spin-coat or spray-coat the electrically conductive coating material.

Electric conducting layer 203 is formed on flexible substrate 204, and is formed upon manufacturing flexible substrate 204. In this regard, however, it is possible to form electric conducting layer 203 by sticking metal coat or Cu tape after forming flexible substrate 204. Electric conducting layer 201 of CMUT chip 20 and electric conducting layer 203 of flexible substrate 204 are electrically connected by electric conducting layer 202. Electric conducting layer 202 is formed using an electrically conductive tape such as a Cu tape or Al tape, or electrically conductive paste, etc. impregnated with Ag-particles or C-particles.

Since electric conducting layer 201 is formed in the manufacturing process of a CMUT wafer as mentioned above, there is no engulfment of extraneous substances into an electric conducting layer while mounting CMUT chip 20 or flexible substrate 204, or during the wire bonding process or the mounting process such as filling process of photo curing resin, thus there is no influence due to application of pressure in the acoustic lens bonding process, which leads to improvement in forming a safe ultrasonic probe.

### (10. Eighth Embodiment)

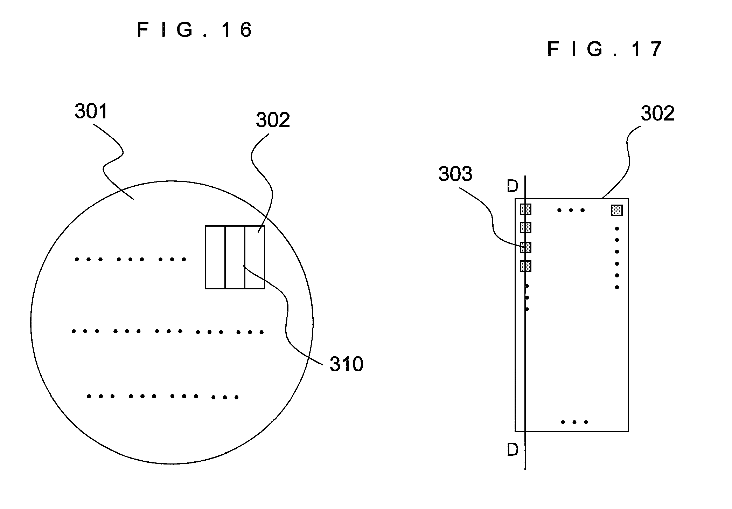

Next, the outline of the relationship between the CMUT wafer and the CMUT chip using Fig. 16 and Fig. 17. Fig. 16 shows the layout of a plurality of CMUT chips 30 in a plane of CMUT wafer 301. The CMUT wafer 301 is manufactured using a wafer of, for example, 8 inches, 6 inches or 12 inches. After completing the manufacture of CMUT wafer 301, it is diced along scribe lines 310, and the respective CMUT chips 302 are segmented.

Fig. 17 is a schematic diagram in which one CMUT chip 302 is enlarged. In CMUT chip 302, electrode pad 303 is formed for electrically connecting to the outside.

### (Ninth Embodiment)

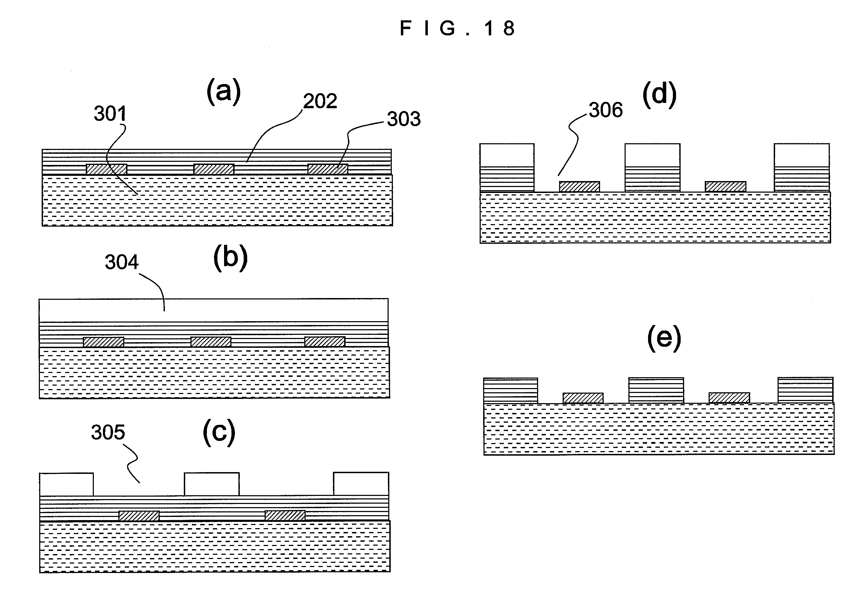

Next, the forming method of an electric conducting layer to CMUT wafer 301 will be described referring to Fig. 18. Fig. 18 shows a D-D cross-section illustrated in Fig. 17. Electric conducting layer 202 is formed by CVD or sputtering on CMUT wafer 301 in which vibratory elements (not shown in the diagram) are completed (a).

Next, photoresist 304 is formed by a spin coater or a spray (b). Next, photoresist aperture 305 is formed in photo resist 304 (c) by the photo lithography method.

Then electric conducting layer aperture 306 of an electrode pad is formed on electric conducting layer 202 by dry etching or wet etching (d). Finally, by removing and cleansing photoresist 304, forming of electric conducting layer 202 and electric conducting layer aperture 306 of electrode pad 303 for securing a conducting path to the outside by the wire bonding are completed.

### (12. Tenth Embodiment)

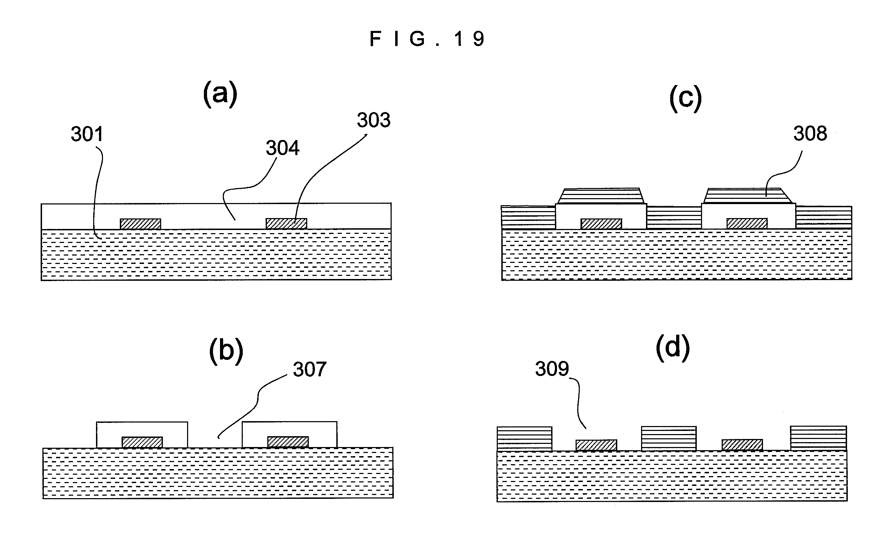

Next, other methods for manufacturing an electric conducting layer to CMUT wafer 301 will be described using Fig. 19. Fig. 19 shows a cross-sectional view of D-D plane shown in Fig. 17. The layer is formed by spin-coating or spray-coating photoresist 304 on CMUT wafer 301 in which a vibration elements (not shown in the diagram) are completed (a) .

Next, photoresist aperture 307 is formed on photoresist 304 using the photo lithography method (b). Then electric conducting layer 308 is formed by CVD or sputtering (c).

Finally, by removing and cleansing photoresist 304, electric conducting layer 308 on electrode pad 303 is removed, and electric conducting layer aperture 309 is formed (d). Compared to the method illustrated in Fig. 18, the advantage of this method is that there is no process for etching an electric conducting layer which makes it easier to form electric conducting layer aperture 309.

However, after removing the last photoresist 304, burrs, etc. tend to remain on electric conducting layer 308 which remains on CMUT wafer 301. This happens when the thickness of electric conducting layer 308 is thicker than the one of photoresist 304, and also when electric conducting layer 308 is thicker than photoresist 304 while electric conducting layer 308 is connected by the edge portion of photoresist 304. In order to prevent burrs from remaining, it is significant that the thickness of photoresist 304 is sufficiently thicker than the one of electric conducting layer 308.

It is known that the ultrasonic probes having configuration that an electric conducting layer is directly formed on a CMUT chip which is described in the first to the tenth embodiments have better acoustic characteristics compared to the conventional ultrasonic probes having the configuration that an electric conducting layer is formed on the side of an acoustic lens. In the case that an electric conducting layer is formed on the side of an acoustic lens, due to an adhesive layer for adhering the acoustic lens existing between the CMUT chip and the electric conducting layer and the distance between the sound source of ultrasonic waves and the electric conducting layer, there are cases that the ultrasonic waves reflected by the electric conducting layer return to the CMUT chip and are further reflected by the CMUT chip, whereby inducing multiple reflections.

In accordance with the present invention, due to the configuration that an electric conducting layer is directly formed on a CMUT chip which makes the electric conducting layer itself to be the sound source to vibrate with transducer elements of ultrasonic waves, it is possible to provide an ultrasonic probe having excellent acoustic characteristic without generating the above-described multiple reflections.

### (13. Other matters)

The above-described embodiments may be appropriately combined to configure an ultrasonic probe and ultrasonic diagnostic apparatus.

Also, in the above-described embodiments, it is preferable to set the thickness of an electric conducting layer as about 0.1µm, and the thickness of an insulating layer as about 1µm. By making the thickness of the insulating layer and the electric conducting layer thin, the influence on the ultrasonic waves transmitted and received by the CMUT chip (influence to or attenuation of pulses and frequency property) can be restrained.

The preferable embodiments of the ultrasonic probe and ultrasonic diagnostic apparatus according to the present invention have been described. However, the present invention is not limited to these embodiments. It is obvious that persons skilled in the art can make various kinds of alterations or modifications within the scope of the technical idea disclosed in this application, and it is understandable that they belong to the technical scope of the present invention.

### Description of Reference Numerals

1: ultrasonic- diagnostic apparatus, 2: ultrasonic probe, 3: transmission/reception separating unit, 4: transmitting unit, 6: bias unit, 8: receiving unit, 10: phasing and adding unit, 12: image processing unit, 14: display unit, 16: controller, 18: operation unit, 20: CMUT chip, 21-1, 21-2, ...: transducer, 22: backing layer, 25: ultrasonic probe cover, 26: acoustic lens, 27: sealant, 28: transducer element, 38 & 41: signal pattern, 40: substrate, 46: upper electrode, 48: lower electrode, 72: flexible substrate, 70, 71 & 90: adhesive layer, 76: electric conducting layer (ground layer), 78 & 78a: insulating film (insulating layer), 84 & 94: ground wire (cable shielding wire), 86 & 183: wires, 88: photo curing resin, 108 & 120: ground, 161, 171, 181, 185, 191 & 195: through hole, 163, 165, 173, 175, 182, 184, 192 & 194: pad terminal, 164, 174 & 193: electric conducting adhesive (anisotropic conducting adhesive sheet), 201, 202, 203 & 308: electric conducting layer, 204: flexible substrate, 301: CMUT wafer, 302: CMUT chip, 303: electrode pad, 304: photoresist, 305 & 307: photoresist aperture, 306 & 309: electric conducting layer aperture.

## Claims

1. An ultrasonic probe comprising:
a CMUT chip having a plurality of transducers that change electromechanical coupling coefficients or sensitivities according to a bias voltage, configured to transmit and receive ultrasonic waves,
an electric conducting layer to be formed on the ultrasonic-wave irradiation side of the CMUT chip:
an acoustic lens to be disposed on the ultrasonic-wave irradiation side of the CMUT chip;
an insulating layer to be formed in the direction opposite from the ultrasonic-wave irradiation side of the acoustic lens; and
a housing unit configured to store the CMUT chip in which the electric conducting layer and the insulating layer are attached with an adhesive and the acoustic lens,
wherein the insulating layer includes one or both of silicon oxide or paraxylene to prevent penetration, and is formed by material which prevents penetration of the adhesive into the adhered portion.

2. The ultrasonic probe according to claim 1, wherein the insulating layer is formed along the inner surface of the acoustic lens.

3. The ultrasonic probe according to claim 1, **characterized in that** the insulating layer is configured by a plurality of insulating films, wherein at least one of the insulating films is formed on the ultrasonic-wave irradiation surface of a CMUT chip, and the remaining insulating layers from the ones formed on the ultrasonic-wave irradiation surface of the CMUT chip are formed along the inner surface of the acoustic lens.

4. The ultrasonic probe according to claim 1, **characterized in that** a ground layer having the ground potential is provided on the ultrasonic-wave irradiation side of the CMUT chip, and the ground layer is connected to a ground wire.

5. The ultrasonic probe according to claim 4, wherein a substrate of the CMUT chip is connected to a ground wire via electric conducting resin from the side direction of a CMUT chip.

6. The ultrasonic probe according to claim 4, wherein:
the CMUT chip has a through hole to electrically conduct the electrode of the CMUT chip to the ultrasonic-wave irradiation surface or to the back surface, and
the electrode of the CMUT chip is connected to a signal pattern of an electric wiring unit via the through hole.

7. The ultrasonic probe according to claim 6, wherein the through hole and a signal pattern of the electric wiring unit are connected by positioning of both of their pad terminals.

8. The ultrasonic probe according to claim 4, wherein:
the CMUT chip has a through hole for electrically conducting a substrate of the CMUT chip to the ultrasonic-wave irradiation surface or to the back surface, and
the substrate of the CMUT chip is connected to a ground wire via the through hole.

9. The ultrasonic probe according to claim 1, comprising a flexible substrate configured to transmit an electrical signal or electric power from the CMUT chip to outside via an electric conducting wire, **characterized in that** an electric conducting layer is formed on the surface of resin material of the flexible substrate.

10. A manufacturing method of an ultrasonic probe comprising:
a CMUT chip having a plurality of transducers that change electromechanical coupling coefficients or sensitivities according to a bias voltage to transmit and receive ultrasonic waves,
an acoustic lens to be disposed on the ultrasonic-wave irradiation side of the CMUT chip;
a backing layer provided on the back surface of the CMUT chip to absorb transmission of the ultrasonic waves;
an electric wiring unit provided on the side surface of the backing layer from the peripheral area of the CMUT chip, in which the signal pattern to be connected to an electrode of the CMUT chip is disposed; and
a housing unit configured to store the CMUT chip, the acoustic lens, the backing layer and the electric wiring unit,
**characterized in** further comprising:
a step that adheres the CMUT chip on the top surface of the backing layer;
a step that adheres the electric wiring unit on the periphery of the top surface of the backing layer;
a step that adheres the electric wiring unit and the CMUT chip via a wire;
a step that fills a sealant around the wire;
a step that forms an electric conducting layer on the ultrasonic-wave irradiation surface of the CMUT chip;
a step that forms an insulating layer on the inner surface of the acoustic lens; and
a step that adheres the acoustic lens on the electric conducting layer formed on the ultrasonic-wave irradiation surface of the CMUT chip.

11. The manufacturing method of an ultrasonic probe according to claim 10 including:
a step that forms a first insulating layer along the ultrasonic-wave irradiation surface of the CMUT chip and the side surface of the flexible substrate and the backing layer; and
a step that forms an electric conducting layer on the first insulating layer.

12. The manufacturing method of an ultrasonic probe according to claim 10, wherein the electric conducting layer on the ultrasonic-wave irradiation surface of the CMUT chip is formed in the wafer condition by CVD or sputtering after forming of transducer elements of an CMUT wafer before segmentizing the wafer into the CMUT chips.

13. The manufacturing method of an ultrasonic probe according to claim 12 **characterized in** forming the electric conducting layer and an electric conducting layer aperture on the CMUT wafer by:
a step that forms an electric conducting layer after forming transducer elements of the CMUT wafer;
a step that forms a photoresist on the electric conducting layer;
a step that provides a photoresist aperture to the photoresist using the photography method;
a step that removes the electric conducting layer of the photoresist aperture portion by etching; and
a step that removes the photoresist.

14. The manufacturing method of an ultrasonic probe according to claim 12 **characterized in** forming an electric conducting layer aperture by:
a step that forms a photoresist after forming transducer elements of the CMUT wafer;
a step that forms a photoresist aperture by the photo lithography method;
a step that forms the electric conducting layer on the photoresist and the photoresist aperture; and
a step that removes the photoresist.

15. An ultrasonic diagnostic apparatus comprising:
an ultrasonic probe configured to transmit/receive ultrasonic waves to/from an object to be examined;
an image processing unit configured to construct an ultrasonic image based on the ultrasonic receiving signals outputted from the ultrasonic probe; and
a display unit configured to display the ultrasonic image,
wherein the ultrasonic probe is as disclosed in claim 1.
